# EUROPEAN PATENT APPLICATION

(11) **EP 4 390 433 A1**
(43) Date of publication of application: **26.06.2024**
(21) Application number: 22214479.2
(22) Date of filing: 19.12.2022
(51) Int. Cl.: G01R 33/563, G01R 33/567, A61B 5/024

(54) **CARDIOVASCULAR MAGNETIC RESONANCE IMAGING USING REMOTE PHOTO PLETHYSMOGRAPHY**

(71) Applicant: Koninklijke Philips N.V., 5656 AG Eindhoven (NL)
(72) Inventor: SPRINGORUM, Rudolf Theodoor, Eindhoven (NL); BLOM, Krelis, Eindhoven (NL); SMINK, Jouke, 5656AG Eindhoven (NL); WUELBERN, Jan Hendrik, Eindhoven (NL)
(74) Representative: Philips Intellectual Property & Standards

(57) **Abstract**

Disclosed herein is a medical system (100, 300) comprising a memory (110) storing machine executable instructions (120) and a computational system (104). Execution of the machine executable instructions causes the computational system to: receive (200) cardiovascular k-space data (122) acquired according to a cardiovascular imaging magnetic resonance imaging protocol and descriptive of a cardiovascular region (326) of a subject (318), wherein the cardiovascular k-space data comprises multiple k-space data portions (126); receive (202) remote photo plethysmograph data (128), wherein the remote photo plethysmograph data is synchronized to an acquisition time of the multiple k-space data portions; determine (204) a recalculated cardiac phase (130) using the remote photo plethysmograph data; map (206) the multiple k-space data portions to a predetermined number of cardiac phases using the recalculated cardiac phase; and reconstruct (208) a cardiovascular image (134) for each of the predetermined number of cardiac phases.

## Description

### FIELD OF THE INVENTION

The invention relates to remote photo plethysmography, in particular to the use of remote photo plethysmography in magnetic resonance imaging.

### BACKGROUND OF THE INVENTION

A large static magnetic field is used by Magnetic Resonance Imaging (MRI) scanners to align the nuclear spins of atoms as part of the procedure for producing images within the body of a patient. This large static magnetic field is referred to as the B0 field.

During an MRI scan, Radio Frequency (RF) pulses generated by one or more transmitter coils cause a called B 1 field. Additionally applied gradient fields and the B 1 field cause perturbations to the effective local magnetic field. RF signals are then emitted by the nuclear spins and detected by one or more receiver coils. These RF signals are recorded as k-space data and are used to reconstruct the MR images, typically using a Fourier or other sparse transform.
Motion of the heart is faster than the acquisition time to acquire a complete set of k-space data. By repeatedly acquiring portions of the k-space data over multiple heart beats, cardiac phase resolved magnetic resonance images may be acquired. A Vector Cardiograph (VCG) signal may be used to trigger when different k-space sampling profiles are used to acquire k-space data.

United States patent publication US 10,993,621 B2 discloses devices and methods to measure and visualize the cardiac and respiratory signal of a human or animal subject during a magnetic resonance imaging (MRI) exam. This includes a video camera compatible with the MRI scanner, a means of transferring the video data away from the MRI scanner, a light source that illuminates the subject, and an algorithm that analyses the video stream and uses small image intensity changes and motion information to extract cardiac signal and respiratory signals of the subject. These methods make it practical to use optical tracking to monitor and correct for cardiac and respiratory motion during MRI, as well as provide basic patient monitoring with no physical contact to the subject.

### SUMMARY OF THE INVENTION

The invention provides for a medical system, a computer program, and a method in the independent claims. Embodiments are given in the dependent claims.

A difficulty in using VCG to control the acquisition of k-space data during cardiovascular magnetic resonance imaging is that a sensor needs to be applied to the subject. A contactless means of measuring the cardiac phase of a subject is remote photo plethysmography. In remote photo plethysmography a camera images facial regions or other areas of exposed skin of the subject and a difference in the appearance of these facial regions or areas of exposed skin is used to provide a remote photo plethysmography (rPPG or PPG) signal. A potential difficulty of using remote photo plethysmography is that the signal may sometimes contain noise and it may sometimes jitter. Embodiments may provide a means of using previously acquired remote photo plethysmograph data to map multiple k-space data portions, that it is synchronized with, to a predetermined number of cardiac phases. This may in some examples provide an improved means of producing cardiovascular magnetic resonance images.

In one aspect the invention provides for a medical system that comprises a memory storing machine-executable instructions and a computational system. Execution of the machine-executable instructions causes the computational system to receive cardiovascular k-space data acquired to a cardiovascular imaging magnetic resonance imaging protocol and the cardiovascular k-space data is descriptive of a cardiovascular region of a subject. The cardiovascular k-space data comprises multiple k-space data portions. Execution of the machine-executable instructions further causes the computational system to receive remote photo plethysmograph data. The remote photo plethysmograph data is synchronized to an acquisition time of the multiple k-space data portions. In other words, the remote photo plethysmograph data is synchronized to the multiple k-space data portions of the cardiovascular k-space data.

Execution of the machine-executable instructions further causes the computational system to determine a recalculated cardiac phase using the remote photo plethysmograph data. The recalculated cardiac phase may also be a prospective cardiac phase. Execution of the machine-executable instructions further causes the computational system to map the multiple k-space data portions to a predetermined number of cardiac phases using the recalculated cardiac phase. Mapping the multiple k-space data portions to the predetermined number of cardiac phases may be different in different examples. In one case it may be a matter of assigning the multiple k-space data portions to different bins representing the predetermined number of cardiac phases.

In other examples, there may be a process of assigning a weighting factor to the different multiple k-space data portions to a particular cardiac phase. For example, if the k-space data is between two of the predetermined number of cardiac phases it may not be advantageous to perform a binning process, in which case there may be a weighting process which assigns a weighting factor to how much the k-space data would affect a particular reconstruction. For example, in this case, if the k-space data portion were between two bins it could be assigned a weighting factor such that it affects these two adjacent cardiac phases but less than a k-space data portion which is better aligned with a particular cardiac phase.

Execution of the machine-executable instructions further causes the computational system to reconstruct a cardiovascular image for each of the predetermined number of cardiac phases using the mapped multiple k-space data according to the cardiovascular imaging magnetic resonance imaging protocol. This embodiment may be beneficial because it may provide for an improved means of aligning k-space data with a remote photo plethysmograph signal.

In another embodiment the cardiovascular k-space data is cardiac k-space data. The cardiovascular imaging magnetic resonance imaging protocol is a cardiac imaging magnetic resonance imaging protocol. The cardiovascular region may include a cardiac region of the subject. This embodiment may be beneficial because the remote photo plethysmograph data may be used as a means of compensating for cardiac motion.

In another embodiment the medical system further comprises a magnetic resonance imaging system. The medical system further comprises a remote photo plethysmograph measurement system configured for measuring the remote photo plethysmograph data from a skin region of the subject during acquisition of the cardiovascular k-space data. The skin region could for example be a facial region, however other regions such as hands, arms, feet, and legs may also be used. The remote photo plethysmograph measurement system may typically be a camera which is used to image a skin region of the subject.

The memory further contains pulse sequence commands that are configured for controlling the magnetic resonance imaging system to acquire the cardiovascular k-space data according to the cardiovascular magnetic resonance imaging protocol. Execution of the machine-executable instructions further causes the computational system to control the magnetic resonance imaging system with the pulse sequence commands to acquire the cardiovascular k-space data. Execution of the machine-executable instructions further causes the computational system to acquire the remote photo plethysmograph data during acquisition of the cardiovascular k-space data.

In some examples the remote photo plethysmograph data may be used to trigger the acquisition of particular sampling patterns for acquiring the cardiovascular k-space data. In other examples, other methods such as an VCG or other technique may be used, and the remote photo plethysmograph data is then acquired and used for later analysis.

In another embodiment the pulse sequence commands are configured to acquire the multiple k-space data portions using a finite number of k-space sampling profiles. Execution of the machine-executable instructions further causes the computational system to calculate a preliminary remote photo plethysmograph signal using the remote photo plethysmograph data. In some examples the preliminary remote photo plethysmograph signal may be a real- time or prospective remote photo plethysmograph signal that is calculated using the remote photo plethysmograph data.

Execution of the machine-executable instructions further causes the computational system to control a selection of the one or more finite number of k-space sampling profiles during acquisition of the multiple k-space data portions using the preliminary remote photo plethysmograph signal. This example may be advantageous because it may provide for a means of triggering the sampling of the different k-space sampling profiles without having to put an electrode or other transducer on the subject.

The remote photo plethysmograph data may be video images of a skin region of the subject. A feature of remote photo plethysmograph data is that it may be somewhat noisy and may be more difficult to determine a cardiac phase than some other techniques such as VCG. In this example, the preliminary remote photo plethysmograph signal is used to trigger the acquisition of the different k-space sampling profiles.

To compensate for instabilities or uncertainty in determining the cardiac phase, the remote photo plethysmograph data is then used later to realign the multiple k-space data portions by mapping them to the predetermined number of cardiac phases. This is then a two-step process where the preliminary remote photo plethysmograph signal is used to control the selection of the finite number of k-space sampling profiles and then a reanalysis of the remote photo plethysmograph data enables a detailed or more accurate mapping of the multiple k-space data portions to the predetermined number of cardiac phases.

In another embodiment the selection of the one or more finite number of k-space sampling profiles is determined in response to detecting a periodic maximum of the preliminary remote photo plethysmograph signal. This embodiment may be beneficial because it may provide for a means of providing a real-time signal or a prospective signal which can be used to predict when the sampling of the k-space sampling profile should begin.

In another embodiment the selection of the one or more finite number of k-space sampling profiles is determined in response to detecting a periodic maximum of a first derivative of the preliminary remote photo plethysmograph signal. This embodiment may be beneficial because it may provide for a means of providing a real-time signal or a prospective signal which can be used to predict when the sampling of the k-space sampling profile should begin.

In another embodiment the selection of the one or more finite number of k-space sampling profiles is determined in response to detecting a periodic minimum of the first derivative of the preliminary remote photo plethysmograph signal. This embodiment may be beneficial because it may provide for a means of providing a real-time signal or a prospective signal which can be used to predict when the sampling of the k-space sampling profile should begin.

In another embodiment the selection of the one or more finite number of k-space sampling profiles is determined by fitting a finite number of harmonic functions to the preliminary remote photo plethysmograph signal. This embodiment may be beneficial because it may provide for a means of providing a real-time signal or a prospective signal which can be used to predict when the sampling of the k-space sampling profile should begin.

In another embodiment the preliminary remote photo plethysmograph signal is calculated using a bandpass filter to remove breathing effects.

In another embodiment the remote photo plethysmograph data comprises a preliminary remote photo plethysmograph signal. The recalculated cardiac phase is determined by analyzing the preliminary remote photo plethysmograph signal. This embodiment may be beneficial because when the preliminary remote photo plethysmograph signal is used to trigger acquisition of particular k-space sampling patterns it may not be clear for example when the maximum of the preliminary remote photo plethysmograph signal is. When the complete preliminary remote photo plethysmograph signal is analyzed, it may then be more accurate to determine the recalculated cardiac phase from it.

In another embodiment the remote photo plethysmograph data comprises a video feed. Execution of the machine-executable instructions further causes the computational system to calculate a recalculated remote photo plethysmograph signal from facial skin patches in the video feed. The recalculated cardiac phase is determined by analyzing the recalculated photo plethysmograph signal. When the complete video feed is available it may for example be possible to more accurately determine the facial skin patches that are used in the video feed. Therefore, the recalculated cardiac phase may be determined more accurately.

In another embodiment the recalculated photo plethysmograph signal is calculated using multiple iterations that refine selection of the facial skin patches. For example, the recalculated photo plethysmograph signal may be refined on different or multiple passes through the video feed that refine the selection of the facial skin patches more accurately and consistently.

In another embodiment the analysis is performed by fitting a preliminary number of high-frequency components to the recalculated photo plethysmograph signal or the preliminary plethysmograph signal. Instead of simply looking at when the maximum of the photo plethysmograph signal occurs, a determined number of high-frequency components can be fit to this and used to determine a cardiac phase.

In another embodiment the analysis is performed by fitting a subject-specific template to the recalculated photo plethysmograph signal or the preliminary plethysmograph signal. The subject-specific template may for example be a particular or pre-measured photo plethysmograph signal that is specific to the subject for which the k-space data is acquired. This for example can be determined by monitoring the remote photo plethysmograph signal for a period of time and determining the subject-specific template. The template for example could be stretched or shifted to fit to the remote plethysmograph data.

In another embodiment the fitting is performed using an over-sampling of the recalculated photo plethysmograph signal or the preliminary plethysmograph signal. When fitting the predetermined number of high-frequency components or the fitting of the subject-specific template, both of these can be fit in a way which enables a determination of the cardiac phase less than the sampling rate. For example, the subject-specific template can be shifted to best match the data and because this template is available it may enable the determination of the cardiac phase more accurately than the sampling rate. The same is true for fitting the predetermined number of high-frequency components.

In another embodiment the analysis is performed by extracting a phase signal from a Hilbert transform of the recalculated plethysmograph signal or the preliminary plethysmograph signal. This may be beneficial because it may provide for a very accurate means of determining the cardiac phase.

In another embodiment the cardiovascular magnetic resonance imaging protocol is CINE magnetic resonance imaging protocol. This may be beneficial because the CINE magnetic resonance imaging may benefit greatly from an improved means of measuring the cardiac phase of a subject and then correcting the k-space data to better fit the predetermined number of cardiac phases.

In another embodiment the cardiovascular magnetic resonance imaging protocol is a phase contrast magnetic resonance imaging protocol. The cardiovascular image for each of the predetermined number of cardiac phases is a phase contrast magnetic resonance image. Execution of the machine-executable instructions further comprises calculating a Q-Flow segmentation of the cardiovascular image for each of the predetermined number of cardiac phases. Execution of the machine-executable instructions further comprises overlaying the Q-Flow segmentation on the cardiovascular image for each of the predetermined number of cardiac phases. This embodiment may be beneficial because it may provide for an improved means of Q-Flow imaging that does not use a contact or sensor which is attached to the subject.

In another aspect the invention provides for a method of medical imaging. The method comprises receiving cardiovascular k-space data acquired according to a cardiovascular imaging magnetic resonance imaging protocol and is descriptive of a cardiovascular region of a subject. The cardiovascular k-space data comprises multiple k-space data portions. The method further comprises receiving remote photo plethysmograph data.

The remote photo plethysmograph data is synchronized to an acquisition time of the multiple k-space data portions. The method further comprises determining a recalculated cardiac phase using the remote photo plethysmograph data. The method further comprises mapping the multiple k-space data portions to a predetermined number of cardiac phases using the recalculated cardiac phase. The method further comprises reconstructing a cardiovascular image for each of the predetermined number of cardiac phases using the mapped multiple k-space data according to the cardiovascular imaging magnetic resonance imaging protocol.

In another aspect the invention provides for a computer program that comprises machine-executable instructions. Execution of the machine-executable instructions causes a computational system to receive cardiovascular k-space data acquired according to a cardiovascular imaging magnetic resonance imaging protocol and the cardiovascular k-space data is descriptive of a cardiovascular region of a subject. The cardiovascular k-space data comprises multiple k-space data portions. The method further comprises receiving remote photo plethysmograph data.

The remote photo plethysmograph data is synchronized to an acquisition time of the multiple k-space data portions. The method further comprises determining a recalculated cardiac phase using the remote photo plethysmograph data. The method further comprises mapping the multiple k-space data portions to a predetermined number of cardiac phases using the recalculated cardiac phase. The method further comprises reconstructing a cardiovascular image for each of the predetermined number of cardiac phases using the mapped multiple k-space data according to the cardiovascular imaging magnetic resonance imaging protocol.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A `computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

`Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some embodiments computer storage may also be computer memory or vice versa.

A `computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.
A `user interface' as used herein is an interface which allows a user or operator to interact with a computer or computer system. A `user interface' may also be referred to as a `human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.
A `hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,
Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Medical imaging data is defined herein as being recorded measurements made by a tomographic medical imaging system descriptive of a subject. The medical imaging data may be reconstructed into a medical image. A medical image id defined herein as being the reconstructed two- or three-dimensional visualization of anatomic data contained within the medical imaging data. This visualization can be performed using a computer.

K-space data is defined herein as being the recorded measurements of radio frequency signals emitted by atomic spins using the antenna of a Magnetic resonance apparatus during a magnetic resonance imaging scan. Magnetic resonance data is an example of tomographic medical image data.

A Magnetic Resonance Imaging (MRI) image or MR image is defined herein as being the reconstructed two-dimensional, three-dimensional, or four-dimensional visualization of anatomic data contained within the magnetic resonance imaging data. This visualization can be performed using a computer.

### BRIEF DESCRIPTION OF THE DRAWINGS

In the following preferred embodiments of the invention will be described, by way of example only, and with reference to the drawings in which:
Fig. 1 illustrates an example of a medical system;
Fig. 2 shows a flow chart which illustrates a method of using the medical system of Fig. 1;
Fig. 3 illustrates a further example of a medical system;
Fig. 4 shows a flow chart which illustrates a method of using the medical system of Fig. 3;
Fig. 5 shows a comparison between a VCG and a preliminary remote plethysmograph signal that illustrates jitter in a cardiac phase determined using the preliminary remote plethysmograph signal;
Fig. 6 shows the comparison between a VCG and a preliminary remote plethysmograph signal of Fig. 5 where a correction to the jitter in the cardiac phase has been determined retrospectively; and
Fig. 7 illustrates a protype of a remote plethysmograph signal.

### DETAILED DESCRIPTION OF EMBODIMENTS

Like numbered elements in these figures are either equivalent elements or perform the same function. Elements which have been discussed previously will not necessarily be discussed in later figures if the function is equivalent.

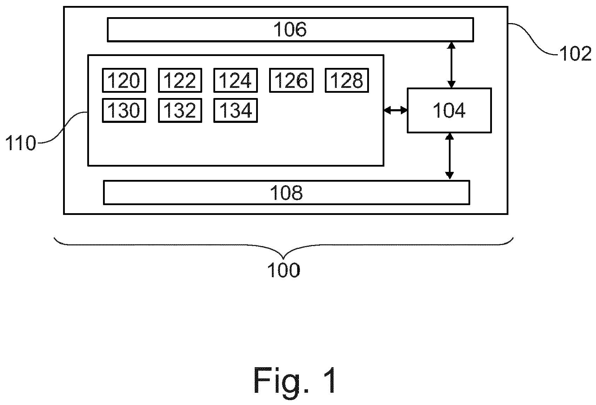

Fig. 1 illustrates an example of a medical system 100. The medical system 100 is shown as comprising a computer 102. The computer 102 may represent one or more computers located at one or more locations. For example, computational tasks may be distributed. The computer 102 is shown as comprising a computational system 104. The computational system 104 may represent one or more computational systems at one or more locations and for example may be one or more computational cores. The computational system 104 is shown as being in communication with an optional hardware interface 106. If other components are present the hardware interface 106 may be used to exchange messages and commands between this other component and the computational system 104. For example, if a magnetic resonance imaging system is part of the medical system 100 then it may be controlled via the hardware interface 106.

The computational system 104 is further shown as being in communication with an optional user interface 108. The user interface 108 may for example be used by an operator to control and operate the medical system 100.

The computational system 104 is further shown as being in communication with a memory 110. The memory 110 is intended to represent various types of memory and storage devices that may be in communication with the computational system 104. In some examples, the memory 110 is a non-transitory storage medium. The medical system 100 could be implemented in different ways. In one example it may be a remote server or cloud-based system which is used to provide reconstruction services for a magnetic resonance imaging system or a radiology department. In other examples, the medical system 100 may be a workstation or computer used by a healthcare professional. In yet other cases the medical system 100 may be incorporated into a medical imaging system such as a magnetic resonance imaging system.

The memory 110 is shown as containing machine-executable instructions 120. The machine-executable instructions enable the computational system 104 to perform various data processing and computational tasks. For example, the machine-executable instructions 120 may enable the computational system to perform image processing and the reconstruction of magnetic resonance images. The memory 110 is further shown as containing cardiovascular k-space data that comprises multiple k-space data portions 126. The memory 110 is further shown as containing remote photo plethysmograph data 128. The remote photo plethysmograph data 128 is synchronized or registered to the various k-space data portions 126.

The memory 110 is shown as storing a recalculated cardiac phase 130 that has been calculated from the remote photo plethysmograph data 128. The memory 110 is further shown as containing a mapping for the various k-space data portions 126 to a predetermined number of cardiac phases 132. In some examples this may be a binning of the k-space data portions 126. In other cases, this may be a value which assigns a weighting to the k-space data portion 126 to a particular cardiac phase. The memory 110 is further shown as containing a cardiovascular image for each of the predetermined number of cardiac phases 134 that has been reconstructed from the various k-space data portions 126 using the mapping 132.

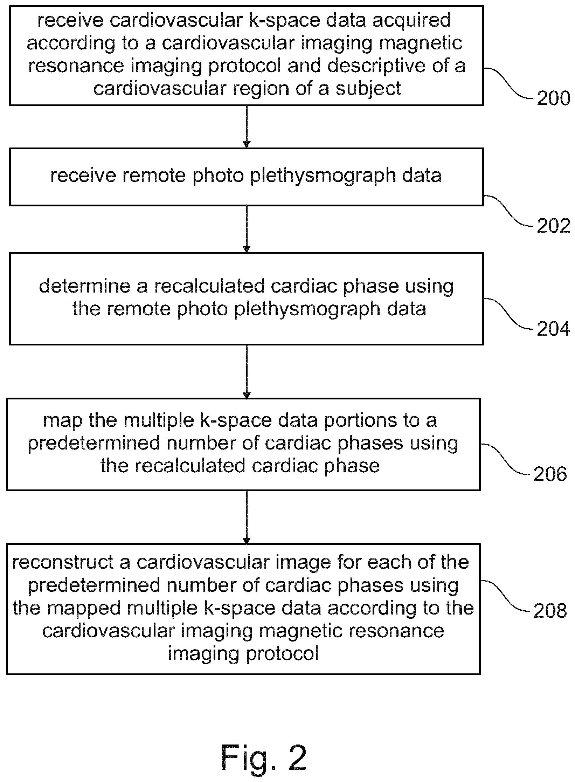

Fig. 2 shows a flowchart which illustrates a method of using the medical system 100 of Fig. 1. First, in step 200, the cardiovascular k-space data 122 is received. The cardiovascular k-space data was acquired according to a cardiovascular imaging magnetic resonance imaging protocol and is descriptive of a cardiovascular region of a subject. Next, in step 202, the remote photo plethysmograph data 128 is received. The remote photo plethysmograph data 128 is synchronized to an acquisition time of the multiple k-space data portions 126. Then, in step 204, the recalculated cardiac phase 130 is determined using the remote photo plethysmograph data 128. In step 206, the multiple k-space data portions 126 are mapped to a predetermined number of cardiac phases using the recalculated cardiac phase 130. Finally, in step 208, the cardiovascular image 134 for each of the predetermined number of cardiac phases is reconstructed from the mapped k-space data portions 126 according to the cardiovascular imaging magnetic resonance imaging protocol.

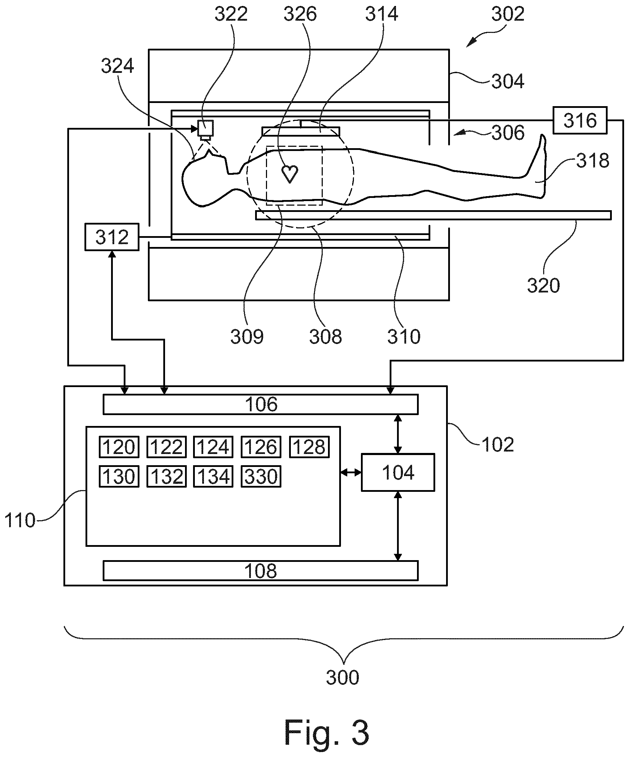

Fig. 3 illustrates a further example of a medical system 300. The medical system 300 is similar to the medical system 100 of Fig. 1 except that it additionally comprises a magnetic resonance imaging system 302. The magnetic resonance imaging system 302 comprises a magnet 304. The magnet 304 is a superconducting cylindrical type magnet with a bore 306 through it. The use of different types of magnets is also possible; for instance, it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. A field of view 309 is shown within the imaging zone 308. The k-space data that is acquired typically acquired for the field of view 309. The region of interest could be identical with the field of view 309 or it could be a sub volume of the field of view 309. A subject 318 is shown as being supported by a subject support 320 such that at least a portion of the subject 318 is within the imaging zone 308 and the field of view 309.

Within the bore 306 of the magnet there is also a set of magnetic field gradient coils 310 which is used for acquisition of preliminary k-space data to spatially encode magnetic spins within the imaging zone 308 of the magnet 304. The magnetic field gradient coils 310 connected to a magnetic field gradient coil power supply 312. The magnetic field gradient coils 310 are intended to be representative. Typically, magnetic field gradient coils 310 contain three separate sets of coils for spatially encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio-frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio-frequency coil 314 is connected to a radio frequency transceiver 316. The radio-frequency coil 314 and radio frequency transceiver 316 may be replaced by separate transmit and receive coils and a separate transmitter and receiver. It is understood that the radio-frequency coil 314 and the radio frequency transceiver 316 are representative. The radio-frequency coil 314 is intended to also represent a dedicated transmit antenna and a dedicated receive antenna. Likewise, the transceiver 316 may also represent a separate transmitter and receivers. The radio-frequency coil 314 may also have multiple receive/transmit elements and the radio frequency transceiver 316 may have multiple receive/transmit channels. The transceiver 316 and the gradient controller 312 are shown as being connected to the hardware interface 106 of the computer system 102.

A remote photo plethysmograph measurement system 322 is shown as being positioned above the subject 318 such that a skin region 324 may be imaged. In this example the remote photo plethysmograph measurement system 322 is a camera system. Often times a remote photo plethysmograph measurement system 322 comprises an illumination source and a camera. An RGB cameras may provide for a superior remote photo plethysmograph signal. However, when imaging the face of a subject 318 the illumination may cause distress. Typically, the remote photo plethysmograph measurement system 322 is constructed using an infra-red camera and an infra-red illumination source. This may provide for a means of illuminating the skin region 324 without disturbing the subject 318.

The memory 110 is further shown as containing the pulse sequence commands 330. The pulse sequence commands 330 are commands or data which may be converted into commands which can control the magnetic resonance imaging system 302 to acquire the measured k-space data 124. The pulse sequence commands 330 are configured to acquire the cardiovascular k-space data 122 using a finite number of k-space sampling profiles. The memory 110 is further shown as containing a preliminary remote plethysmograph signal 322 that is calculated from the remote photo plethysmograph data 128. The preliminary remote photo plethysmograph signal 332 is calculated as the remote photo plethysmograph data 128 is acquired. There may for example be a delay of a predetermined number of samples of the remote photo plethysmograph data 128.

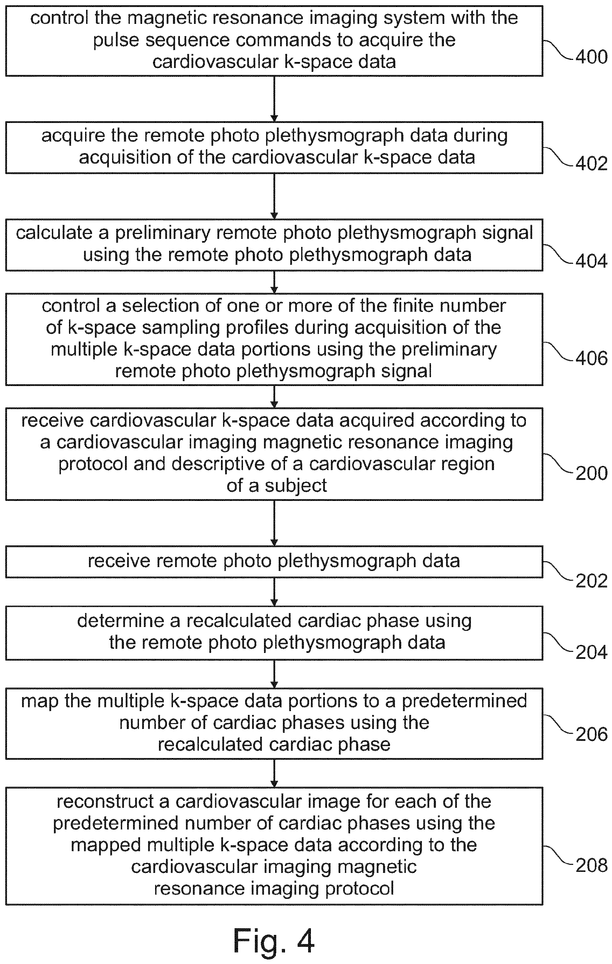

Fig. 4 shows a flowchart which illustrates a method of using the medical system 300 of Fig. 3. First, in step 400, the magnetic resonance imaging system 302 is controlled with the pulse sequence commands 330 to acquire the cardiovascular k-space data 122. Next, in step 402, the remote photo plethysmograph data 128 is acquired during acquisition of the cardiovascular k-space data 122. There is a timing synchronization or registration between the remote photo plethysmograph data 128 and the various k-space data portions 126 of the cardiovascular k-space data 122. Next, in step 404, the preliminary remote photo plethysmograph signal 332 is calculated using the remote photo plethysmograph data 128. As was mentioned, this is done immediately and there may be a delay in several samples of the remote photo plethysmograph data 128 in the preliminary remote photo plethysmograph signal 332. However, the preliminary remote photo plethysmograph signal 332 may be considered to be a real-time signal.

Next, in step 406, a selection of one or more of the finite number of k-space sampling profiles is controlled during acquisition of the multiple k-space portions 126 using the preliminary remote photo plethysmograph signal 332. This may for example be done so that for each of the predetermined number of cardiac phases the k-space sampling profiles have each been sampled at least once. After step 406 is performed, steps 200, 202, 204, 206, and 208 are performed as was illustrated in Fig. 2.

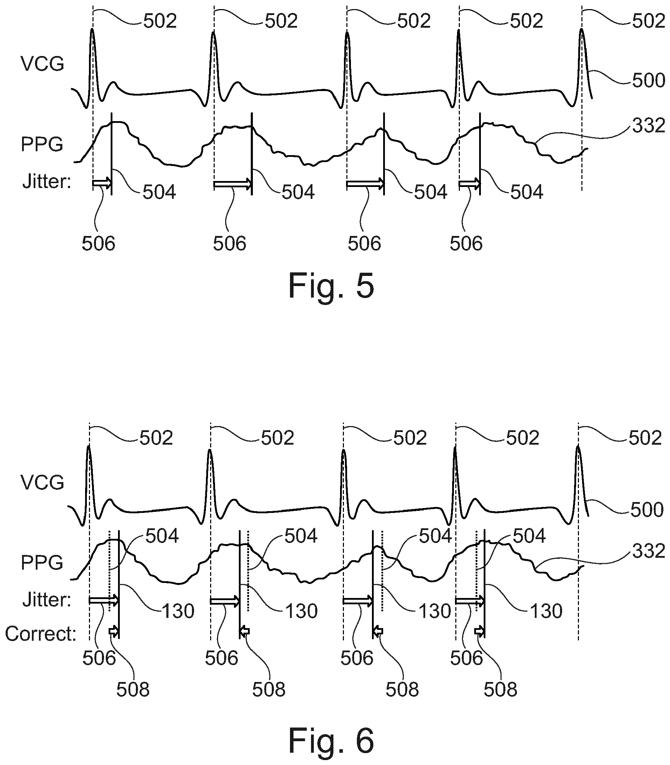

Fig. 5 compares a VCG signal and a real-time acquired preliminary remote photo plethysmograph signal 332. The VCG signal provides a very clear cardiac phase 502. The cardiac phase from the remote photo plethysmograph signal 332 can be used to provide the preliminary cardiac phase 504. However, it is observed that this preliminary cardiac phase 504 has a relatively large jitter 506 caused by noise in the signal and also an uncertainty as to when the maximum of the preliminary remote photo plethysmograph signal 332 is. This illustrates the observed variations in peak detection for a real-time optimized algorithm for rPPG. Compared to the VCG, the real-time optimized detection on PPG data exhibits significant jitter, a variation in VCG to PPG measured delay time, illustrated by the black arrows.

Fig. 6 illustrates how this jitter 506 can be corrected. Fig. 6 illustrates the same curves 500 332. In this case the recalculated cardiac phase 130 has been determined from the preliminary remote photo plethysmograph signal 332 after it has been completely acquired. This allows a correction 508 to be determined to accurately determine the recalculated cardiac phase 130. It can be seen in this case that the jitter 506 has been corrected for. The recalculated cardiac phase 130 is then accurate enough to provide a basis for mapping the k-space data portions 126 to the predetermined number of cardiac phases.

During acquisition the peaks detected in real-time in (Fig. 5) are still used to synchronize the acquisition. The peaks found by the quality optimized algorithm in (Fig. 6) are then used to map the data to their corresponding cardiac phase and reconstruct the final images.

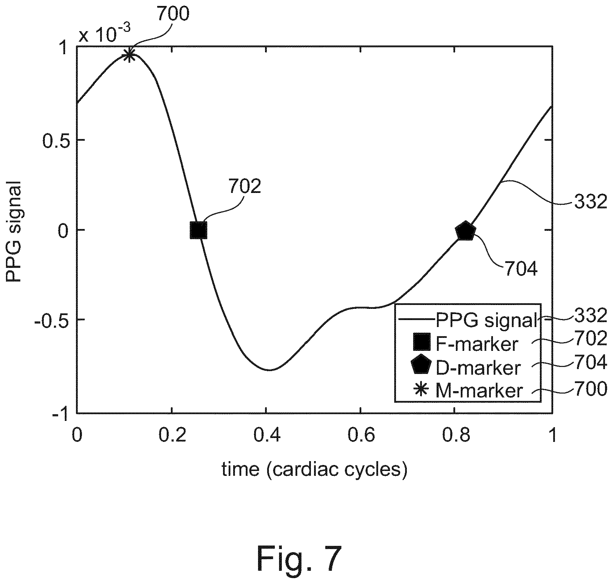

Reanalysis of the acquired or preliminary PPG signal may include one or more of the following techniques:
1. Interpolation of the PPG signal to achieve accuracy which exceeds original sampling time.
2. Using other characteristic markers within the cardiac cycle (Fig. 3) of the PPG trace (e.g. minimum, minimum or maximum of the first derivative, etc.)
3. Improved marker detection based on template matching, where the template of the PPG cardiac cycle (Fig. 7) is derived from all PPG cycles recorded for the current individual/patient.

Fig. 7 illustrates a prototype of a PPG representation of a cardiac cycle, it illustrates how a remote plethysmograph signal 332 may be analyzed and provide for a more accurate determination of the recalculated cardiac phase 130. Fig. 7 shows a curve with the preliminary remote photo plethysmograph signal 332. A first marker may be the maximum 700. The first derivative of the curve 332 may also be used. 702 indicates the minimum of the first derivative and marker 704 indicates the maximum of the first derivative. The combination of these three markers may provide for an improved means of determining the recalculated cardiac phase 130.

During reconstruction, the multiple k-space data portions are sorted following their relative position within the cardiac cycle. Images are reconstructed from chunks collected over multiple beats that best represent the relative position within the cardiac cycle using a sliding window reconstruction.

When the detected triggers do not accurately represent the exact same phase in each cardiac cycle used for acquisition, due to a poor detection of the peaks, the reconstructed images may suffer from temporal blurring.

A more precise retrospectively applied signal analysis results in an improved accuracy of the found trigger markers. This could then be used to re-map all data chunks to their more precise positions in the cardiac cycle, thereby reducing the temporal motion blurring. As the jitter during the acquisition might lead to missing K-space data during part of the cardiac cycle, a slight oversampling (5%-10%) may be applied to accommodate for these variations, filling in the blank spots for which no K-space data is available.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: computer
- 104: computational system
- 106: optional hardware interface
- 108: optional user interface
- 110: memory
- 120: machine executable instructions
- 122: cardiovascular k-space data
- 126: k-space data portion
- 128: remote photo plethysmograph data
- 130: recalculated cardiac phase
- 132: mapping to predetermined number of cardiac phases
- 134: cardiovascular image for each of the predetermined number of cardiac phases
- 200: receive cardiovascular k-space data acquired according to a cardiovascular imaging magnetic resonance imaging protocol and descriptive of a cardiovascular region of a subject
- 202: receive remote photo plethysmograph data
- 204: determine a recalculated cardiac phase using the remote photo plethysmograph data
- 206: map the multiple k-space data portions to a predetermined number of cardiac phases using the recalculated cardiac phase
- 208: reconstruct a cardiovascular image for each of the predetermined number of cardiac phases using the mapped multiple k-space data according to the cardiovascular imaging magnetic resonance imaging protocol
- 300: medical system
- 302: magnetic resonance imaging system
- 304: magnet
- 306: bore of magnet
- 308: imaging zone
- 309: field of view
- 310: magnetic field gradient coils
- 312: magnetic field gradient coil power supply
- 314: radio-frequency coil
- 316: transceiver
- 318: subject
- 320: subject support
- 322: remote photo plethysmograph measurement system
- 324: skin (facial) region
- 326: cardiovascular region
- 330: pulse sequence commands
- 332: preliminary remote plethysmograph signal
- 400: control the magnetic resonance imaging system with the pulse sequence commands to acquire the cardiovascular k-space data
- 402: acquire the remote photo plethysmograph data during acquisition of the cardiovascular k-space data
- 404: calculate a preliminary remote photo plethysmograph signal using the remote photo plethysmograph data
- 406: control a selection of one or more of the finite number of k-space sampling profiles during acquisition of the multiple k-space data portions using the preliminary remote photo plethysmograph signal
- 500: VCG signal
- 502: cardiac phase from VCG signal
- 504: preliminary cardiac phase
- 506: jitter
- 508: correction

## Claims

1. A medical system (100, 300) comprising:
- a memory (110) storing machine executable instructions (120);
- a computational system (104), wherein execution of the machine executable instructions causes the computational system to:
- receive (200) cardiovascular k-space data (122) acquired according to a cardiovascular imaging magnetic resonance imaging protocol and descriptive of a cardiovascular region (326) of a subject (318), wherein the cardiovascular k-space data comprises multiple k-space data portions (126);
- receive (202) remote photo plethysmograph data (128), wherein the remote photo plethysmograph data is synchronized to an acquisition time of the multiple k-space data portions;
- determine (204) a recalculated cardiac phase (130) using the remote photo plethysmograph data;
- map (206) the multiple k-space data portions to a predetermined number of cardiac phases using the recalculated cardiac phase; and
- reconstruct (208) a cardiovascular image (134) for each of the predetermined number of cardiac phases using the mapped multiple k-space data according to the cardiovascular imaging magnetic resonance imaging protocol.

2. The medical system of claim 1, wherein the medical system further comprises:
- a magnetic resonance imaging system (302); and
- a remote photo plethysmograph measurement system (322) configured for measuring the remote photo plethysmograph data from a skin region (324) of the subject during acquisition of the cardiovascular k-space data;
wherein the memory further contains pulse sequence commands (330) configured for controlling the magnetic resonance imaging system to acquire the cardiovascular k-space data according to the cardiovascular magnetic resonance imaging protocol;
wherein execution of the machine executable instructions further causes the computational system to:
- control (400) the magnetic resonance imaging system with the pulse sequence commands to acquire the cardiovascular k-space data; and
- acquire (402) the remote photo plethysmograph data during acquisition of the cardiovascular k-space data.

3. The medical system of claim 2, wherein the pulse sequence commands are configured to acquire the multiple k-space data portions using a finite number of k-space sampling profiles, wherein execution of the machine executable instructions further causes the computational system to:
- calculate (404) a preliminary remote photo plethysmograph signal (332) using the remote photo plethysmograph data; and
- control (406) a selection of one or more of the finite number of k-space sampling profiles during acquisition of the multiple k-space data portions using the preliminary remote photo plethysmograph signal.

4. The medical system of claim 3, wherein the selection of the one or more of the finite number of k-space sampling profiles is determined in response to any one of the following:
- detecting a periodic maximum (700) of the preliminary remote photo plethysmograph signal;
- detecting a periodic maximum (704) of a first derivative of the preliminary remote photo plethysmograph signal;
- detecting a periodic minimum (702) of the first derivative of the preliminary remote photo plethysmograph signal;
- detecting a preliminary cardiac phase determined by fitting a finite number of harmonic functions to the preliminary remote photo plethysmograph signal, and wherein the preliminary remote photo plethysmograph signal is preferably calculated using a bandpass filter to remove breathing effects; and
- combinations thereof.

5. The medical system of claim 1 or 2, wherein the remote photo plethysmograph data comprises a preliminary remote photo plethysmograph signal, and wherein the recalculated cardiac phase is determined by analyzing the preliminary remote photo plethysmograph signal.

6. The medical system of any one of claims 1 through 4, wherein the remote photo plethysmograph data comprises a video feed, wherein execution of the machine executable instructions further causes the computational system to calculate a recalculated photo plethysmograph signal from facial skin patches (324) in the video feed, wherein the recalculated cardiac phase is determined by analyzing the recalculated photo plethysmograph signal.

7. The medical system of claim 6, wherein the recalculated photo plethysmograph signal is calculated using multiple iterations that refine selection of the facial skin patches.

8. The medical system of claim 5, 6, or 7, wherein the analysis is performed by fitting a predetermined number of high frequency components to the recalculated photo plethysmograph signal or the preliminary plethysmograph signal.

9. The medical system of claim 5, 6, or 7, wherein the analysis is performed by fitting a subject specific template to the recalculated photo plethysmograph signal or the preliminary remote plethysmograph signal.

10. The medical system of claim 8 or 9, wherein the fitting is performed using over-sampling of the recalculated photo plethysmograph signal or the preliminary plethysmograph signal.

11. The medical system of claim 5, 6, or 7, wherein the analysis is performed by extracting a phase signal from a Hilbert transform of the recalculated photo plethysmograph signal or the preliminary plethysmograph signal.

12. The medical system of any one of the preceding claims, wherein the cardiovascular magnetic resonance imaging protocol is a CINE magnetic resonance imaging protocol.

13. The medical system of any one of claims 1 to 11, wherein the cardiovascular magnetic resonance imaging protocol is a phase contrast magnetic resonance imaging protocol, wherein the cardiovascular image for each of the predetermined number of cardiac phases is a phase contrast magnetic resonance image, wherein execution of the machine executable instructions further comprises:
- calculating a q-flow segmentation of the cardiovascular image for each of the predetermined number of cardiac phases; and
- overlaying the q-flow segmentation on the cardiovascular image for each of the predetermined number of cardiac phases.

14. A method of medical imaging, wherein the method comprises:
- receiving (200) cardiovascular k-space data (122) acquired according to a cardiovascular imaging magnetic resonance imaging protocol and descriptive of a cardiovascular region (326) of a subject (318), wherein the cardiovascular k-space data comprises multiple k-space data portions;
- receiving (202) remote photo plethysmograph data (128), wherein the remote photo plethysmograph data is synchronized to an acquisition time of the multiple k-space data portions;
- determining (204) a recalculated cardiac phase (130) using the remote photo plethysmograph data;
- mapping (206) the multiple k-space data portions to a predetermined number of cardiac phases using the recalculated cardiac phase; and
- reconstructing (208) a cardiovascular image (134) for each of the predetermined number of cardiac phases using the mapped multiple k-space data according to the cardiovascular imaging magnetic resonance imaging protocol.

15. A computer program comprising machine executable instructions, wherein execution of the machine executable instructions causes a computational system to:
- receive (200) cardiovascular k-space data (122) acquired according to a cardiovascular imaging magnetic resonance imaging protocol and descriptive of a cardiovascular region (326) of a subject (318), wherein the cardiovascular k-space data comprises multiple k-space data portions (126);
- receive (202) remote photo plethysmograph data (128), wherein the remote photo plethysmograph data is synchronized to an acquisition time of the multiple k-space data portions;
- determine (204) a recalculated cardiac phase (130) using the remote photo plethysmograph data;
- map (206) the multiple k-space data portions to a predetermined number of cardiac phases using the recalculated cardiac phase; and
- reconstruct (208) a cardiovascular image (134) for each of the predetermined number of cardiac phases using the mapped multiple k-space data according to the cardiovascular imaging magnetic resonance imaging protocol.
